# EUROPEAN PATENT APPLICATION

(11) **EP 4 201 954 A1**
(43) Date of publication of application: **28.06.2023**
(21) Application number: 21217058.3
(22) Date of filing: 22.12.2021
(51) Int. Cl.: C07K 14/725, C12N 5/0783, A61P 1/00, C07K 16/28

(54) **PROTEINS AND T-CELLS INVOLVED IN CHRONIC INFLAMMATORY DISEASES**

(71) Applicant: Christian-Albrechts-Universität zu Kiel, 24118 Kiel (DE)
(72) Inventor: Rosati, Elisa, 24116 Kiel (DE); Franke, Andre, 24119 Kronshagen (DE); Bacher, Petra, 24105 Kiel (DE); Mamedov, Ilgar, 11-1-29 Moskau (RU)
(74) Representative: Eisenführ Speiser

(57) **Abstract**

A protein comprising an amino acid sequence according to SEQ ID NO: 1.

## Description

The present invention relates to proteins comprising an amino acid sequence according to a specific motive. The invention further relates to a nucleotide sequence encoding for such an amino acid sequence and a T-cell comprising the protein according to the invention. Further, the invention relates to the use of a protein according to the invention or a T-cell according to the invention or an mRNA according to the invention in the research for a medicament for treatment of a chronic inflammatory disease or in the development of an antibody directed against the protein and/or the T-cell and/or the mRNA according to the invention.

The invention further relates to an antibody directed against an epitope of the protein according to the invention.

### State of the art

Dysregulated T cell reactions against intestinal antigens are considered to be a causal or driving factor for chronic inflammatory diseases, especially inflammatory bowel diseases (IBD). For IBD and for other inflammatory and autoimmune diseases, e.g. multiple sclerosis or diabetes, the excessive immune response may be directed against common, still unknown, disease-driving antigens. In genetically predisposed hosts, antigens derived from a dysbiotic microbiome may come into contact with T cells through the damaged intestinal epithelial barrier, which may lead to an uncontrolled and dysregulated immune response. As such, it may be possible to identify specific disease-associated T cells within the peripheral blood and/or at the intestinal inflammation site of IBD patients.

Although the two main forms of IBD, namely Crohn's disease (CD) and ulcerative colitis (UC) are often referred to as generally "IBD", they are two independent diseases with different disease progression and characteristics. CD may develop in any part of the gastrointestinal tract, mostly in the small intestine, while UC develops in the colon only. Both diseases are T-cell mediated, different T cell subsets may but do not have to play a role in disease pathogenesis and progression. Markers distinguishing the two diseases exist but are limited, sometimes resulting in incorrect diagnosis. Further insights differentiating the two forms would help in better understanding, diagnosis and treatment of the diseases.

Most alpha/beta T cells are characterized by a unique T-cell receptor (TCR), formed by an alpha and beta chain. T cells recognize antigenic peptides presented by the major histocompatibility complex (MHC). Upon recognition of an antigen via the TCR, T cells may expand clonally, resulting in a population of cells with identical TCRs ("clonotypes"). The TCR repertoire, defined as the collection of unique TCRs, is extremely variable, with up to 10¹⁶ unique clonotypes present in a single individual. Therefore, the composition of the TCR repertoire, i.e. the diversity of different TCRs or the expansion of individual clonotypes, can provide important insights into disease-associated alterations of the T cell reaction.

In general, there is a need for the identification of disease-associated T-cell clonotypes in chronic inflammatory disease patients, especially in IBD patients, which could be an important step towards (i) understanding the contribution of TCR repertoire changes to diseases' pathophysiology; (ii) the identification of pathogenic T cells of interest; (iii) the identification of pathogenic T cells' cognate antigens and (iv) the development of new diagnostic and therapeutic strategies. Previous studies identified expanded clonotypes in IBD intestinal samples compared to blood samples and correlated certain TCRs with disease recurrence after surgery.

In view of the state of the art, it was an object of the present invention to provide means for research and development of diagnostic and therapeutic strategies for chronic inflammatory diseases, especially for IBD.

In a first inspect of the invention this object is solved by a protein comprising an amino acid sequence according to SEQ ID and NO: 1.

Surprisingly, as result of the inventors' research there is an amino acid sequence motive according to SEQ ID NO: 1 that is associated with the occurrence of inflammatory diseases, especially involved in the occurrence of IBD and more prevalently involved in Crohn's disease (CD).

As used herein a "protein" refers to a polypeptide with at least 60, preferably at least 80 and more preferably at least 100 amino acids.

According to the invention a protein according to the invention is preferred wherein the amino acid in position 4 of SEQ ID NO: 1 is selected from the group consisting of N, S, R, T and I, preferably consisting of N, S and R and/or wherein the amino acid in position 5 of SEQ ID NO: 1 is selected from the group consisting of R, V, L, F, M, I, H, S, T, A, P and G.

Alternatively, or additional preferred is a protein according to the invention, wherein the amino acid in position 7 of SEQ ID NO: 1 is A or S, wherein A is preferred.

Preferably the protein according to the invention is a protein, wherein the amino acid sequence according to SEQ ID NO: 1 is selected from the group consisting of amino acid sequences SEQ ID NO: 2 to 295.

More preferably the protein according to the invention is a TCR.

As used herein, a "T-cell receptor", or "TCR", is a molecule found on the surface of T-cells, or T-lymphocytes, that is responsible for recognizing antigenic molecules, more preferably peptides or/and lipids, bound to mayor histocompatibility complex (MHC) or MHC-like molecules. The TCR is a heterodimer. There are two types of TCRs: αβTCR consisting of an alpha (α) chain and a beta (β) chain (∼95% of TCRs in humans) and γδTCR consisting of a gamma and delta chain (∼5% of TCRs in humans). When the TCR engages with the antigen and MHC complex, the T-lymphocyte is activated through signal transduction, mediated by associated enzymes, co-receptors, specialized adaptor molecules, and activated or inactivated or released transcription factors.

The TCR α and β chains are both composed of a variable part and a constant part. The diversity of the TCR is based mainly on genetic recombination of the DNA encoded segments, originating from the VDJ genes, in individual T cells. The process of recombination ultimately results in novel TCR amino acids sequences that allow for the recognition of antigens.

As used herein the "T-cell receptor", and "TCR" relates both to naturally occurring T-cell receptors and recombinantly expressed T-cell receptors.

As used herein a T-cell, or a lymphocyte, is a type of lymphocytes (a subtype of white blood cells) that plays a central role in cell mediated immunity. T-cells can be distinguished from other lymphocytes, such as B-cells and natural killer cells, by the presence of a T-cell receptor on the cell surface. They are called T-cells because they mature in the thymus from thymocytes.

A protein of the invention is preferred wherein the amino acid sequence according to SEQ ID NO: 1 is comprised in the alpha chain of the TCR, more preferably in the CDR3 region.

As used herein the CDR3 region is the most variable region of a TCR. The CDR3 region of the TCR alpha chain is positioned at the junction between the V and the J TCR genes and is the result of the VDJ recombination process during T cell maturation in the thymus. The CDR3 region is the most studied region of the TCR, because has the highest contacts with the antigen.

According to the invention it is preferred that the amino acid sequence according to SEQ ID NO: 1 (or preferred sequence according to the invention) is encoded by the genes *TRAV12-1* and/or *TRAJ6.*

Surprisingly the inventors could show that proteins according to the invention, especially the preferred proteins according to the invention were found in (human) patients affected by an inflammatory disease, especially an inflammatory bowel disease (IBD) and more especially Crohn's disease in a significant higher number as compared to healthy control or non-inflammatory disease control individuals. In addition, the cumulative abundance of the proteins according to the invention was also significantly higher in CD patients' blood.

Further, the presence and abundance of proteins according to the invention did not seem to correlate with any specific MHC-allele. Nor was found a correlation between the abundance of those proteins and other clinical parameters such as age, gender, disease severity or medication history.

In a second aspect of the invention the above object is solved by a T cell, comprising the protein of the invention. Preferably such a T cell is a human T cell.

More preferably the T cell according to the invention is a CD4⁺ cell and/or a CD8⁺ cell and/or a CD161+ cell, preferably a CD8⁺ cell.

CD4, CD8 and CD161 are proteins expressed on the surface of T lymphocytes or/and other immune cells. These proteins work as co-stimulatory molecules in different cellular signals, including but not limited to the TCR response.

A "CD4⁺ cell", as used herein, is a cell that expresses the CD4 protein on its surface or expresses the mRNA encoding for the CD4 protein.

A "CD8⁺ cell", as used herein, a cell that expresses the CD8 protein on its surface or expresses the mRNA encoding for the CD8 protein.

A "CD161⁺ cell", as used herein, is a cell that expresses the CD161 protein on its surface or expresses the mRNA encoding for the CD161 protein.

The T cells according to the invention are a good basis for further research for medicaments and antibodies to be used in the treatment inflammatory diseases especially in the treatment of IBD and more especially in the treatment of CD.

In a third aspect of the invention the above object is solved by a nucleotide encoding for an amino acid sequence according to sequence ID NO: 1, preferably according to any of amino acid sequences SEQ ID NO: 2 to 295, wherein the nucleotide sequence more preferably is selected from the DNA sequences SEQ ID NO: 296 to 1205, or an mRNA sequence encoded by a DNA sequence according to any of DNA sequence SEQ ID NO: 296 to 1205.

Preferably a nucleotide sequence according to the invention is an mRNA sequence.

A fourth aspect of the present invention is a use of a protein according to the invention, or of a T cell according to the invention or of an mRNA according to the invention in the research for a medicament for treatment of a chronic inflammatory disease preferably inflammatory bowel disease (IBD).

More preferred is a use according to the invention, wherein the chronic inflammatory disease is IBD, preferably Crohn's disease (CD) or ulcerative colitis (UC), preferably CD, more preferably ileal CD and/or ileocolonic CD.

A fifth aspect according to the invention is a use of a protein according to the invention or a use of a T cell according to the invention or a use of an mRNA according to the invention in the development of an antibody directed against the protein and/or the T cell or the mRNA.

A preferred use according to the invention is a use wherein the antibody is directed against an epitope, comprising at least a part of the amino acid sequence according to SEQ ID NO: 1, wherein preferably the amino acid sequence according to SEQ ID NO: 1 is further defined as described above.

A sixth aspect of the invention is an antibody directed against an epitope, comprising at least a part of the amino acid sequence according to SEQ ID NO: 1, wherein preferably the amino acid sequence according to SEQ ID NO: 1 is further defined as above described and/or wherein the antibody is a monoclonal antibody.

A "monoclonal antibody" as used herein comprises antibodies with the same monovalent affinity, thus binding to the same epitope.

Certainly, in the sense of the present invention the antibodies according to the invention are preferably specific antibodies wherein "specific" means the antibody is able to bind the target epitope according to the invention and/or the antibody can discriminate between that epitope and another similar or dissimilar epitope.

### Examples

### MATERIAL AND METHODS

### Example 1

### Whole blood sample collection

Phenotypic details for this collection are described in Table 1.

CD patient collection: BioCrohn is a non-interventional prospective registry of Crohn's disease patients undergoing biologics treatment which was established by the German "Competence Network IBD", Kiel, Germany in 2008. Samples included in this study were collected prior to the start of anti-TNF therapy (Infliximab).

To evaluate disease activity, the Harvey-Bradshaw Index (HBI) was used. This measure was employed to distinguish between inactive (0≤HBI≤4: remission) and active state of the disease (5≤HBI≤7: mild activity, 8≤HBI≤16: moderate activity, 17≤HBI≤∞: severe activity)(42), as described from the Canadian society of intestinal research. In total, whole blood samples of 109 CD patients were available for analysis. All patients had active disease at sampling time. Response to anti-TNF therapy was defined as a steroid-independent change from active disease (HBI > 4 at baseline) to an inactive state of the disease (HBI < 4 at 6 months after start of therapy). Accordingly, non-response to anti-TNF therapy was defined as the disease being consistently active at both time points under investigation (HBI > 4 at baseline and 6 months).

UC patient collection: blood samples from UC patients of the "whole blood collection" were recruited as part of a larger panel of patients with IBD, recruited in the Comprehensive Centre for Inflammation Medicine (CCIM), at the University Hospital Schleswig-Holstein, Campus Kiel (Germany). Samples for the present study were randomly selected among the available samples stored in the PopGen biobank. In total, whole blood samples of 36 UC patients were available.

Healthy control collection: blood samples were obtained from the PopGen control sample, consisting of a community-based sample from the Kiel area (Northern Germany) and of blood donors from the University Hospital Schleswig-Holstein, Campus Kiel(43). In total, 1316 individuals were recruited at baseline (between 2005 and 2007) and were re-examined at the second examination cycle; between 2010 and 2012; n=952)(44, 45). Participants filled-in questionnaires on health and nutrition, received a basic clinical examination and provided blood samples for research. For the present analyses, individuals with major diseases such as cancer, neurological and inflammatory diseases were excluded. In total, 99 whole blood samples were available. These samples were matched to the CD patients in regard to age and sex.

The study was approved by the local ethics committees (Ethics votes: A156/03 v. 29.7.2010, A156/03 - 3/15 v. 21.1.16, A156/03-2/13, A161/08). All study participants gave their written informed consent.

2.5 ml of blood were drawn and stored in PAXgene tubes (Qiagen), following the manufacturer's instructions. Samples were stored at -80°C. Total RNA was isolated using the PAXgene blood miRNA kit (Qiagen) with automatic isolation using the Qiacube machine. 1,200 ng of total RNA were used for

**Table 1: Whole blood collection phenotype information. Information about disease phenotype was provided via the Montreal classification. Information on the disease activity was available through the Harvey-Bradshaw index (HBI) for CD patients and though the colitis activity index (CAI) and partial Mayo score for UC patients. Information about steroid therapy at sampling time was also provided. "NA" corresponds to "information not available".**

| | **Sample group** | | |
|---|---|---|---|
| | **CD** | **UC** | **Healthy** |
| **Basic phenotype** | | | |
| Samples | 109 | 36 | 99 |
| Gender, Male/Female (%) | 43/65 (40/60%) | 21/15 (58/42%) | 41/58 (41/59%) |
| Age, mean±std | 40±12 | 37±13 | 48±13 |
| **Montreal classification** | | | |
| *Age at diagnosis in years* | | | |
| A1, <16) (%) | 7 (6%) | - | - |
| A2, 17-40 (%) | 74 (70%) | - | - |
| A3, >40 (%) | 25 (24%) | - | - |
| *Behaviour* | | | |
| B1, inflammatory (%) | 51 (47%) | - | - |
| B2, stricturing (%) | 21 (19%) | - | - |
| B3, penetrating (%) | 36 (34%) | - | - |
| *Location* | | | |
| L1, ileal (%) | 29 (27%) | - | - |
| L2, colonic (%) | 23 (21%) | - | - |
| L3, ileocolonic (%) | 56 (52%) | - | - |
| *Disease extent* | | | |
| E1, proctitis (%) | - | 3 (3%) | - |
| E2, left-sided UC (%) | - | 16 (47%) | - |
| E3, extensive UC (%) | - | 15 (44%) | - |
| Info not available (NA) | 1 | 2 | - |
| **Disease activity** | | | |
| HBI, mean±std | 11±5 | - | - |
| Mild activity, 5≤HBI≤7 (%) | 37 (34%) | - | - |
| Moderate activity, 8≤HBI≤16 (%) | 57 (53%) | - | - |
| Severe activity, 17≤HBI≤∞ (%) | 14 (13%) | - | - |
| Partial Mayo score, mean±std | - | 3.3±2 | - |
| CAI, mean±std | - | 6.7±4.3 | - |
| Info not available (NA) | 1 | 0 | - |
| **Medications** | | | |
| Steroids, yes/no (%) | 7/102 (6/94%) | 14/22 (39/61%) | - |
| Treatment naive yes/no/NA (%) | 77/14/18 (70/13/17%) | 0/0/36 (0/0/100%) | - |
| Responders to anti-TNF yes/no/NA (%) | 46/60/3 (42/55/3%) | | |

### Example 2

### Surgery sample collection

Samples were collected in collaboration with the Clinic for General and Thoracic Surgery of the University Hospital Schleswig-Holstein, Kiel, Germany. It is important to notice that for CRC tissue samples, macroscopically normal, non-tumour tissue was utilized. Sample details are provided in Table 2.

Sample collection included (1) one 9 ml whole blood EDTA tube from which PBMCs were isolated by density gradient centrifugation (Ficoll, GE Healthcare) and (2) resected intestinal mucosa. Tissues were cut into pieces of approximately 30-50 mg, snap-frozen and stored at -80°C. Details on the number of collected samples and intestinal resection location for each patient are available in Table 2, together with patients' basic phenotypic information.

Total RNA was isolated using the RNeasy mini kit from Qiagen following the manufacturer's instructions. Up to 3,200 ng of total RNA were used for TCR library preparation. Details on the sorted T cell populations from additional surgery samples are described in the supplementary materials and methods as well as supplementary table 1.

**Table 2: Surgery collection phenotype information. Information about the numbers of collected samples for each tissue is provided together with information about intestinal location of the sample and about therapy with steroids or immunosuppressants at time of surgery.**

| | **CD** | **UC** | **CRC** |
|---|---|---|---|
| **Basic phenotype** | | | |
| Samples | 11 | 13 | 13 |
| Gender, Male/Female (%) | 4/7 (36/64%) | 7/6 (54/46%) | 8/5 (62/38%) |
| Age, mean±std | 40±15 | 52±16 | 69±12 |
| **Sample types** | | | |
| Intestinal samples | 11 | 13 | 9 |
| Mesenteric lymph nodes | 4 | 5 | 0 |
| PBMCs | 11 | 12 | 12 |
| Intestine + PBMCs datasets | 11 | 12 | 8 |
| **Resected tissue location** | | | |
| *Large intestine* | 4 | 13 | 13 |
| *Small intestine* | 7 | 0 | 0 |
| **Medications** | | | |
| Steroids, yes/no/NA | 4/5/2 | 7/6/0 | 0/0/13 |
| Immunosuppressants, yes/no/NA | 5/5/1 | 2/10/1 | 0/0/13 |

### Example 3

### TCR library preparation and sequencing

Starting from total RNA, molecular-barcoded TCR cDNA libraries were prepared as previously described in M. V. Pogorelyy et al., Persisting fetal clonotypes influence the structure and overlap of adult human T cell receptor repertoires. PLOS Computational Biology 13, e1005572-e1005572 (2017*),* with minor modifications for both TCRα and TCRβ chains. Briefly, cDNA synthesis was performed using SMARTScribe reverse transcriptase (Clon-tech, Takara) using primers for the TCRα and TCRβ constant region. A unique molecular identifier (UMI), and a sample barcode of 6 nucleotides, were introduced via template-switching. cDNA synthesis was carried out for 60 minutes at 42°C. cDNA was then treated with Uracil DNA-Glycosylase (UDG, from New England Biolabs) and incubated for 30 min at 37°C. Samples were subsequently purified with the QIAquick PCR purification kit (Qiagen) and eluted in 50 or 100 µl deionized water. Purified cDNA was then amplified with 2 consecutive PCRs, respectively 18 and 12 cycles, with purification after each PCR using MagSi-NGSprep Plus (MagnaMedics). Illumina compatible adapters and sample-specific barcodes were added during the second PCR. Quality and concentration of the libraries were measured with TapeStation D1000 (Agilent) and Qubit (Thermofisher). Libraries were pooled using 5 ng per library and sequenced on Illumina HiSeq2500 with a single-index Rapid Run of 2×100 bp (whole blood) or on Illumina NovaSeq 6000 SP 2×150 bp flow cell (surgery). Custom sequencing primers were added to the Illumina primers.

For the sorted surgery collection samples, libraries were prepared using human TCR profiling kit (MiLaboratory), according to the manufacturers' protocol. Libraries were sequenced on Illumina MiSeq 2×150 bp or NovaSeq 6000 SP 2×150 bp.

### Example 4

### TCR data pre-processing

PCR and sequencing error correction were performed through identification and selection of unique molecular identifiers using the software MiGEC, version 1.2.6. Filtered sequences were aligned on a TCR gene reference, clonotypes were identified, grouped and CDR3 sequence was identified using the software MiXCR, version 2.1.1 (first sample collection: blood collection) or version 3.0.14 (second collection: surgery collection). Clonotype tables containing clonotype counts, frequencies, CDR3 nucleotide and amino acid sequences and V(D)J genes were obtained and used for further analysis.

A total of 444 samples were analysed for bulk TCR repertoire.

### Example 5

### TCR data analysis

For statistical analysis, the Wilcoxon-Mann-Whitney U test was used unless otherwise indicated. Samples with less than 200 identified unique clonotypes were excluded from the analysis. TCRs found at a count inferior to 2 (singletons) or encoding for out of frame sequences or TCRs containing stop codons, were also excluded from further analyses.

Proportion analysis of TCR groups (CDR3length_Vgene_Jgene): For each disease group (CD, UC, healthy), the total number of TCR sequences being part of each TCR group (CDR3length_Vgene_Jgene) was calculated and divided by the number of total TCR sequences present in each disease group in orderto normalize for differences in TCR number among disease groups. The proportion of TCRs of each TCR group was then compared between disease groups, either CD against UC, or against healthy controls, for both TCR alpha and TCR beta.

Fisher test on identified TCR groups of interest: using specific CDR3 and VJ gene combinations identified through the analysis of repertoire proportions (12aa_TRAV1-2_TRAJ33, 15aa_TRAV12-1_TRAJ6), we performed one-sided Fisher's exact test on the single TCRs of these groups to identify TCR alpha clonotypes respectively decreased (12aa_TRAV1-2_TRAJ33) or increased (15aa_TRAV12-1_TRAJ6) in CD patients versus healthy individuals, from the whole blood and surgery collections. We applied multiple testing correction through the Benjamini-Hochberg (BH) method(49), in order to control expected proportion of false discoveries amongst the rejected hypotheses (false discovery rate, FDR).

Analysis on specific sequences: TCR sequences from the identified group of CDR3length = 15 amino acids, V gene = TRAV12-1 and J gene = TRAJ6 that were found to be more abundant in CD patients as compared to controls in the whole blood sample collection were used for the logo plot which defined the specific clonotype motif CVV**A*GGSYIPTF (SEQ ID NO: 1). All sequences of the selected TCR group, carrying the motif were selected for further analysis. For each sample, the number of sequences according to the invention present in the sample as well as the sum of relative abundances (cumulative abundance) of CAIT sequences according to the invention were calculated.

The results can be found in the table 3 (amino acid sequences) and in the sequence protocol (SEQ ID NO: 2-295 (amino acid sequences) and SEQ ID NO: 296-1205 (DNA sequences)).

As can be seen from table 3 there are polypeptide sequences with higher occurrences. It has to been noted that according to the invention a polypeptide sequence is the more preferred the higher the occurrence is according to table 3.

**Table 3 TCR sequences**

| **SEQ ID NO** | **Occurrencies** | **Sequence** |
|---|---|---|
| 2 | 144 | CVVNLASGGSYIPTF |
| 3 | 93 | CVVNGASGGSYIPTF |
| 4 | 88 | CVVNSASGGSYIPTF |
| 5 | 77 | CVVNIASGGSYIPTF |
| 6 | 77 | CVVNPASGGSYIPTF |
| 7 | 75 | CVVNRASGGSYIPTF |
| 8 | 72 | CVVSLASGGSYIPTF |
| 9 | 62 | CVVNMASGGSYIPTF |
| 10 | 55 | CVVTLASGGSYIPTF |
| 11 | 49 | CVVNTASGGSYIPTF |
| 12 | 43 | CVVRLASGGSYIPTF |
| 13 | 42 | CVVRHASGGSYIPTF |
| 14 | 38 | CVVNVASGGSYIPTF |
| 15 | 37 | CVVSHASGGSYIPTF |
| 16 | 36 | CVVRVASGGSYIPTF |
| 17 | 34 | CVVRSASGGSYIPTF |
| 18 | 33 | CVVIPASGGSYIPTF |
| 19 | 31 | CVVNKASGGSYIPTF |
| 20 | 30 | CVVRGASGGSYIPTF |
| 21 | 30 | CVVTPASGGSYIPTF |
| 22 | 29 | CVVNAASGGSYIPTF |
| 23 | 29 | CVVNFASGGSYIPTF |
| 24 | 24 | CVVSSASGGSYIPTF |
| 25 | 23 | CVVNQASGGSYIPTF |
| 26 | 21 | CVVRTASGGSYIPTF |
| 27 | 19 | CVVIHASGGSYIPTF |
| 28 | 19 | CVVNEASGGSYIPTF |
| 29 | 19 | CVVNHASGGSYIPTF |
| 30 | 19 | CVVRRASGGSYIPTF |
| 31 | 18 | CVVILASGGSYIPTF |
| 32 | 18 | CVVRIASGGSYIPTF |
| 33 | 16 | CVVRAASGGSYIPTF |
| 34 | 16 | CVVSPASGGSYIPTF |
| 35 | 16 | CVVSTASGGSYIPTF |
| 36 | 16 | CVVTHASGGSYIPTF |
| 37 | 16 | CVVTSASGGSYIPTF |
| 38 | 16 | CVVTVASGGSYIPTF |
| 39 | 15 | CVVNMAAGGSYIPTF |
| 40 | 15 | CVVSVASGGSYIPTF |
| 41 | 15 | CVVTAASGGSYIPTF |
| 42 | 14 | CVVKGASGGSYIPTF |
| 43 | 12 | CVVNGAPGGSYIPTF |
| 44 | 12 | CVVNIAAGGSYIPTF |
| 45 | 12 | CVVNMATGGSYIPTF |
| 46 | 12 | CVVPHASGGSYIPTF |
| 47 | 12 | CVVSAASGGSYIPTF |
| 48 | 12 | CVVTFASGGSYIPTF |
| 49 | 11 | CVVREASGGSYIPTF |
| 50 | 11 | CVVSFASGGSYIPTF |
| 51 | 11 | CVVSGASGGSYIPTF |
| 52 | 11 | CVVSIASGGSYIPTF |
| 53 | 10 | CVVIAASGGSYIPTF |
| 54 | 10 | CVVISASGGSYIPTF |
| 55 | 10 | CVVNMAGGGSYIPTF |
| 56 | 9 | CVVFHASGGSYIPTF |
| 57 | 9 | CVVRQASGGSYIPTF |
| 58 | 9 | CVVSRASGGSYIPTF |
| 59 | 8 | CVVIGASGGSYIPTF |
| 60 | 8 | CVVLHASGGSYIPTF |
| 61 | 8 | CVVNIATGGSYIPTF |
| 62 | 8 | CVVNYASGGSYIPTF |
| 63 | 8 | CVVRFASGGSYIPTF |
| 64 | 8 | CVVTLAGGGSYIPTF |
| 65 | 7 | CVVAHASGGSYIPTF |
| 66 | 7 | CVVIRASGGSYIPTF |
| 67 | 7 | CVVNGAAGGSYIPTF |
| 68 | 7 | CVVNIAGGGSYIPTF |
| 69 | 7 | CVVRMASGGSYIPTF |
| 70 | 7 | CVVTIASGGSYIPTF |
| 71 | 6 | CVVKHASGGSYIPTF |
| 72 | 6 | CVVKSASGGSYIPTF |
| 73 | 6 | CVVMGASGGSYIPTF |
| 74 | 6 | CVVNDASGGSYIPTF |
| 75 | 6 | CVVNRAPGGSYIPTF |
| 76 | 6 | CVVTGASGGSYIPTF |
| 77 | 5 | CVVALASGGSYIPTF |
| 78 | 5 | CVVDHASGGSYIPTF |
| 79 | 5 | CVVIFASGGSYIPTF |
| 80 | 5 | CVVIIASGGSYIPTF |
| 81 | 5 | CVVIVASGGSYIPTF |
| 82 | 5 | CVVKPASGGSYIPTF |
| 83 | 5 | CVVKRASGGSYIPTF |
| 84 | 5 | CVVKTASGGSYIPTF |
| 85 | 5 | CVVNLAAGGSYIPTF |
| 86 | 5 | CVVNSAAGGSYIPTF |
| 87 | 5 | CVVNSAGGGSYIPTF |
| 88 | 5 | CVVRKASGGSYIPTF |
| 89 | 5 | CVVSQASGGSYIPTF |
| 90 | 4 | CVVFLASGGSYIPTF |
| 91 | 4 | CVVFSASGGSYIPTF |
| 92 | 4 | CVVKAASGGSYIPTF |
| 93 | 4 | CVVKLASGGSYIPTF |
| 94 | 4 | CVVMHASGGSYIPTF |
| 95 | 4 | CVVNLAGGGSYIPTF |
| 96 | 4 | CVVNMAPGGSYIPTF |
| 97 | 4 | CVVNRAAGGSYIPTF |
| 98 | 4 | CVVNRATGGSYIPTF |
| 99 | 4 | CVVNVAAGGSYIPTF |
| 100 | 4 | CVVNVATGGSYIPTF |
| 101 | 4 | CVVNWASGGSYIPTF |
| 102 | 4 | CVVSKASGGSYIPTF |
| 103 | 4 | CVVSQAAGGSYIPTF |
| 104 | 4 | CVVTDASGGSYIPTF |
| 105 | 4 | CVVTYASGGSYIPTF |
| 106 | 3 | CVVGLASGGSYIPTF |
| 107 | 3 | CVVGTASGGSYIPTF |
| 108 | 3 | CVVIDASGGSYIPTF |
| 109 | 3 | CVVITASGGSYIPTF |
| 110 | 3 | CVVKVASGGSYIPTF |
| 111 | 3 | CVVLLASGGSYIPTF |
| 112 | 3 | CVVNGATGGSYIPTF |
| 113 | 3 | CVVNIAPGGSYIPTF |
| 114 | 3 | CVVNKAAGGSYIPTF |
| 115 | 3 | CVVNLARGGSYIPTF |
| 116 | 3 | CVVNSAVGGSYIPTF |
| 117 | 3 | CVVNTAGGGSYIPTF |
| 118 | 3 | CVVNVAGGGSYIPTF |
| 119 | 3 | CVVTQASGGSYIPTF |
| 120 | 3 | CVVTTASGGSYIPTF |
| 121 | 3 | CVVYHASGGSYIPTF |
| 122 | 3 | CVVYLASGGSYIPTF |
| 123 | 2 | CVVADASGGSYIPTF |
| 124 | 2 | CVVAQASGGSYIPTF |
| 125 | 2 | CVVASASGGSYIPTF |
| 126 | 2 | CVVATASGGSYIPTF |
| 127 | 2 | CVVFGASGGSYIPTF |
| 128 | 2 | CVVHHASGGSYIPTF |
| 129 | 2 | CVVHVASGGSYIPTF |
| 130 | 2 | CVVINASGGSYIPTF |
| 131 | 2 | CVVIQASGGSYIPTF |
| 132 | 2 | CVVKEASGGSYIPTF |
| 133 | 2 | CVVKIASGGSYIPTF |
| 134 | 2 | CVVKKASGGSYIPTF |
| 135 | 2 | CVVKMASGGSYIPTF |
| 136 | 2 | CVVKRAGGGSYIPTF |
| 137 | 2 | CVVLGASGGSYIPTF |
| 138 | 2 | CVVLPASGGSYIPTF |
| 139 | 2 | CVVLSASGGSYIPTF |
| 140 | 2 | CVVLTASGGSYIPTF |
| 141 | 2 | CVVMPASGGSYIPTF |
| 142 | 2 | CVVNEAAGGSYIPTF |
| 143 | 2 | CVVNIALGGSYIPTF |
| 144 | 2 | CVVNIARGGSYIPTF |
| 145 | 2 | CVVNMAEGGSYIPTF |
| 146 | 2 | CVVNPATGGSYIPTF |
| 147 | 2 | CVVNSATGGSYIPTF |
| 148 | 2 | CVVPFASGGSYIPTF |
| 149 | 2 | CVVPPASGGSYIPTF |
| 150 | 2 | CVVPRASGGSYIPTF |
| 151 | 2 | CVVPSASGGSYIPTF |
| 152 | 2 | CVVPTASGGSYIPTF |
| 153 | 2 | CVVRLAGGGSYIPTF |
| 154 | 2 | CVVRPASGGSYIPTF |
| 155 | 2 | CVVRTARGGSYIPTF |
| 156 | 2 | CVVRVAAGGSYIPTF |
| 157 | 2 | CVVRYASGGSYIPTF |
| 158 | 2 | CVVSDASGGSYIPTF |
| 159 | 2 | CVVSGAVGGSYIPTF |
| 160 | 2 | CVVSLAGGGSYIPTF |
| 161 | 2 | CVVVHASGGSYIPTF |
| 162 | 2 | CVVVLASGGSYIPTF |
| 163 | 1 | CVVAFASGGSYIPTF |
| 164 | 1 | CVVAGASGGSYIPTF |
| 165 | 1 | CVVAPASGGSYIPTF |
| 166 | 1 | CVVAVASGGSYIPTF |
| 167 | 1 | CVVAWASGGSYIPTF |
| 168 | 1 | CVVDDASGGSYIPTF |
| 169 | 1 | CVVDQASGGSYIPTF |
| 170 | 1 | CVVEQASGGSYIPTF |
| 171 | 1 | CVVFAASGGSYIPTF |
| 172 | 1 | CVVFLAGGGSYIPTF |
| 173 | 1 | CVVFQARGGSYIPTF |
| 174 | 1 | CVVFQASGGSYIPTF |
| 175 | 1 | CVVFRASGGSYIPTF |
| 176 | 1 | CVVFTASGGSYIPTF |
| 177 | 1 | CVVFVASGGSYIPTF |
| 178 | 1 | CVVGDASGGSYIPTF |
| 179 | 1 | CVVG IASGGSYI PTF |
| 180 | 1 | CVVGRASGGSYIPTF |
| 181 | 1 | CVVGVAAGGSYIPTF |
| 182 | 1 | CVVGVASGGSYIPTF |
| 183 | 1 | CVVHDASGGSYIPTF |
| 184 | 1 | CVVHEASGGSYIPTF |
| 185 | 1 | CVVHLASGGSYIPTF |
| 186 | 1 | CVVHQASGGSYIPTF |
| 187 | 1 | CVVHYASGGSYIPTF |
| 188 | 1 | CVVIPATGGSYIPTF |
| 189 | 1 | CVVISAPGGSYIPTF |
| 190 | 1 | CVVIYASGGSYIPTF |
| 191 | 1 | CVVKAAAGGSYIPTF |
| 192 | 1 | CVVKDASGGSYI PTF |
| 193 | 1 | CVVKFASGGSYIPTF |
| 194 | 1 | CVVKGAGGGSYIPTF |
| 195 | 1 | CVVKGAPGGSYIPTF |
| 196 | 1 | CVVKLAVGGSYIPTF |
| 197 | 1 | CVVKNASGGSYIPTF |
| 198 | 1 | CVVKSAGGGSYIPTF |
| 199 | 1 | CVVKTAAGGSYIPTF |
| 200 | 1 | CVVKTAGGGSYIPTF |
| 201 | 1 | CVVKVAGGGSYIPTF |
| 202 | 1 | CVVKYASGGSYIPTF |
| 203 | 1 | CVVLGAAGGSYIPTF |
| 204 | 1 | CVVLVASGGSYIPTF |
| 205 | 1 | CVVMAASGGSYIPTF |
| 206 | 1 | CVVMKAEGGSYIPTF |
| 207 | 1 | CVVMLASGGSYIPTF |
| 208 | 1 | CVVMRASGGSYIPTF |
| 209 | 1 | CVVMTASGGSYIPTF |
| 210 | 1 | CVVNAAAGGSYIPTF |
| 211 | 1 | CVVNAAPGGSYIPTF |
| 212 | 1 | CVVNAARGGSYIPTF |
| 213 | 1 | CVVNAATGGSYIPTF |
| 214 | 1 | CVVNEAGGGSYIPTF |
| 215 | 1 | CVVNEATGGSYIPTF |
| 216 | 1 | CVVNGAGGGSYIPTF |
| 217 | 1 | CVVNGAQGGSYIPTF |
| 218 | 1 | CVVNGARGGSYIPTF |
| 219 | 1 | CVVNGAVGGSYIPTF |
| 220 | 1 | CVVNIAEGGSYIPTF |
| 221 | 1 | CVVNIAIGGSYIPTF |
| 222 | 1 | CVVNKAKGGSYIPTF |
| 223 | 1 | CVVNKAPGGSYIPTF |
| 224 | 1 | CVVNKATGGSYIPTF |
| 225 | 1 | CVVNLAVGGSYIPTF |
| 226 | 1 | CVVNMALGGSYIPTF |
| 227 | 1 | CVVNMAQGGSYIPTF |
| 228 | 1 | CVVNMARGGSYIPTF |
| 229 | 1 | CVVNNAPGGSYIPTF |
| 230 | 1 | CVVNNARGGSYIPTF |
| 231 | 1 | CVVNNASGGSYIPTF |
| 232 | 1 | CVVNPALGGSYIPTF |
| 233 | 1 | CVVNPAPGGSYIPTF |
| 234 | 1 | CVVNQAAGGSYIPTF |
| 235 | 1 | CVVNQATGGSYIPTF |
| 236 | 1 | CVVNRAGGGSYIPTF |
| 237 | 1 | CVVNSAEGGSYIPTF |
| 238 | 1 | CVVNSAKGGSYIPTF |
| 239 | 1 | CVVNTAAGGSYIPTF |
| 240 | 1 | CVVNTAKGGSYIPTF |
| 241 | 1 | CVVNTAPGGSYIPTF |
| 242 | 1 | CVVNTARGGSYIPTF |
| 243 | 1 | CVVNVAPGGSYIPTF |
| 244 | 1 | CVVNWAAGGSYIPTF |
| 245 | 1 | CVVNWAGGGSYIPTF |
| 246 | 1 | CVVPEASGGSYIPTF |
| 247 | 1 | CVVPGASGGSYIPTF |
| 248 | 1 | CVVPLASGGSYIPTF |
| 249 | 1 | CVVPLATGGSYIPTF |
| 250 | 1 | CVVPPAPGGSYIPTF |
| 251 | 1 | CVVPVASGGSYIPTF |
| 252 | 1 | CVVQGASGGSYIPTF |
| 253 | 1 | CVVQHASGGSYIPTF |
| 254 | 1 | CVVQLASGGSYIPTF |
| 255 | 1 | CVVQSASGGSYIPTF |
| 256 | 1 | CVVRDASGGSYIPTF |
| 257 | 1 | CVVREAWGGSYIPTF |
| 258 | 1 | CVVRFARGGSYIPTF |
| 259 | 1 | CVVRGAEGGSYIPTF |
| 260 | 1 | CVVRHATGGSYIPTF |
| 261 | 1 | CVVRQAGGGSYIPTF |
| 262 | 1 | CVVRRAAGGSYIPTF |
| 263 | 1 | CVVRSATGGSYIPTF |
| 264 | 1 | CVVRTAQGGSYIPTF |
| 265 | 1 | CVVRVAGGGSYIPTF |
| 266 | 1 | CVVSEASGGSYIPTF |
| 267 | 1 | CVVSGAQGGSYIPTF |
| 268 | 1 | CVVSLAAGGSYIPTF |
| 269 | 1 | CVVSLAKGGSYIPTF |
| 270 | 1 | CVVSMASGGSYIPTF |
| 271 | 1 | CVVSPAPGGSYIPTF |
| 272 | 1 | CVVSQAGGGSYIPTF |
| 273 | 1 | CVVSRAMGGSYIPTF |
| 274 | 1 | CVVSSAVGGSYIPTF |
| 275 | 1 | CVVSTATGGSYIPTF |
| 276 | 1 | CVVTEASGGSYIPTF |
| 277 | 1 | CVVTGAAGGSYIPTF |
| 278 | 1 | CVVTIAEGGSYIPTF |
| 279 | 1 | CVVTNAPGGSYIPTF |
| 280 | 1 | CVVTPAPGGSYIPTF |
| 281 | 1 | CVVTRASGGSYIPTF |
| 282 | 1 | CVVTSARGGSYIPTF |
| 283 | 1 | CVVTSATGGSYIPTF |
| 284 | 1 | CVVTTAGGGSYIPTF |
| 285 | 1 | CVVVFASGGSYIPTF |
| 286 | 1 | CVVVGASGGSYIPTF |
| 287 | 1 | CVVVMASGGSYIPTF |
| 288 | 1 | CVVVRASGGSYIPTF |
| 289 | 1 | CVVWGASGGSYIPTF |
| 290 | 1 | CVVWPASGGSYIPTF |
| 291 | 1 | CVVWTASGGSYIPTF |
| 292 | 1 | CVVYFASGGSYIPTF |
| 293 | 1 | CVVYGASGGSYIPTF |
| 294 | 1 | CVVYMAPGGSYIPTF |
| 295 | 1 | CVVYPASGGSYIPTF |

### Example 6

### Single-cell analysis

Blood was drawn from three CD patients, 2 females and 1 male of age 29, 49 and 36 year old respectively, who were shown to have high abundance of TCRs according to the invention during bulk TCR analysis, as well as from three age and sex matched healthy controls. PBMCs were isolated by gradient centrifugation from EDTA blood tubes. CD3+ cells were enriched by MACS (Miltenyi Biotec).

For single-cell analysis, CD3+ (VioBle) CD45RO+ (PeVio770) memory T cells were sorted in CD8+ (VioGreen) and CD4+ (APCVio770) subpopulations. Cells were removed from the sorting chamber into pre-coated low-bind collection tubes and centrifuged for 5 min at 400x g, 4°C. Cells were re-suspended in PBS plus 0,04% BSA to a concentration of 1000 cells/ul.

Single-cell suspensions were loaded on a Chromium Chip G (10x Genomics) according to manufacturer's instructions for processing with the Chromium Next GEM Single Cell 5' Library and Gel Bead Kit v1.1. Depending on the number of cells available for each patient, a maximum of 20,000 cells were loaded for each reaction. TCR single-cell libraries were subsequently prepared from the same cells with the Chromium Single Cell V(D)J Enrichment Kit, Human T Cell. Libraries were sequenced on the Illumina NovaSeq 6000 machine with 2×100 bp for gene expression, aiming for 50,000 reads per cell and 2×150 bp and 5000 reads per cell for TCR libraries.

Single-cell T cell receptor repertoire clonotype tables were generated using the VDJ command of the Cellranger software, version 3.1.0 from 10xGenomics and using the VDJ reference version 2.0.0. Clonotype tables were filtered in order to include only cells which passed quality filtering in the gene expression analysis. In addition, clonotypes were stringently filtered for possible doublets by removing clonotypes (i) found in 1 cell only and containing more than 1 TCR alpha and 1 TCR beta (ii) containing more than 1 TCR alpha and no TCR beta sequence (iii) containing more than 1 TCR beta and no TCR alpha sequence (iv) containing more than 2 TCR alpha or more than 2 TCR beta sequences.

Gene expression matrices were generated through the COUNT command of Cellranger v3.1.0. from 10xGenomics and using the reference GRCh38 version 3.0. Data were analyzed using the Seurat v3.2.3 R package(24). Cells with more than 400 but less than 3500 detected RNA features per cell and less than 8% mitochondrial RNA were retained for further analysis. Only genes present in at least 1% of the cells were considered. Also, only cells with a detected TCR were used. TCR VDJ genes were removed from gene expression counts to allow for an unbiased analysis. Data from multiple individuals were merged and batch effect correction by experimental day and sequencing run was performed using the Harmony package. 20 dimensions were used for performing principal component analysis (PCA) and uniform manifold approximation and projection (UMAP) in 3 dimensions. Clusters were identified using 0.4 resolution. Positive and negative marker genes were identified using Seurat FindMarkers function and the MAST method, considering only genes found in at least 25% of cells of each cluster.

Cells according to the invention were defined from TCR alpha of 15aa, TRAV12-1 and TRAJ6. Proportion of CAIT cells carrying certain TRBV genes was calculated on the number of unique CAIT clonotypes identified, independently from the number of cells carrying each clonotypes and originated by the same original clonotype by clonal expansion.

### Example 7

### Sample collection and cell staining for flow cytometry immunophenotyping

Whole blood samples were collected in 9 ml EDTA tubes.

Crohn's disease patients: samples were collected in collaboration with the Comprehensive Center for Inflammation Medicine (CCIM) of the University Hospital Schleswig-Holstein, Kiel, Germany. A total of 20 CD patients were recruited. Sample details are provided in Supplementary Table 5.

Healthy human blood: 16 samples were collected from volunteer workers at the University Hospital Schleswig-Holstein, Kiel, Germany.

Peripheral blood mononuclear cells (PBMCs) were isolated by standard density gradient (Biocoll, Biochrom, Berlin Germany) and freshly used for staining. PBMCs were washed with PBS + 2mM EDTA + 0.5% BSA (PEB buffer) and then stained with a cell surface antibody cocktail for CD3 (OKT3, Biolegend), CD4 (M-T466, Miltenyi Biotec), CD8 (REA734, Miltenyi Biotec), CD14 (TÜK4, Miltenyi Biotec), CD20 (LT20, Miltenyi Biotec), CD161 (DX12, BD Biosciences), IL-18Rα/CD218a (REA1095, Miltenyi Biotec), TRAV1-2 (F1, Invitrogen), TRAV12-1 (REA179, Miltenyi Biotec), at room temperature for 30 min in the dark. Cells were washed again and resuspended in 300uL of PEB buffer. Propidium Iodide (Miltenyi Biotec) was used to exclude dead cells. Data were acquired on a LSR Fortessa (BD Bioscience, San Jose, CA, USA). Flow cytometry data were analyzed using FlowJo (Treestar, Ashland, OR, USA) software.

**Table 5: Immunophenotyping blood sample collection phenotype information. Information about disease phenotype was provided via de Montreal classification. Disease score was available through the Harvey-Bradshaw index (HBI) for CD patients. NA corresponds to "information not available".**

| | **Sample group** | |
|---|---|---|
| | **CD** | **Healthy** |
| **Basic phenotype** | | |
| Samples | 20 | 16 |
| | 11/9 | 7/9 |
| Gender, M/F (%) | (55/45%) | (43/57%) |
| Age, mean±std | 44±18 | 31±8 |

| **Montreal classification** | | |
|---|---|---|
| *Age at diagnosis in years* | | |
| A1, <16) (%) | 3(15%) | - |
| A2, 17-40 (%) | 11 (55%) | - |
| A3, >40 (%) | 4 (20%) | - |
| Info not available (NA) | 2 | |
| *Behaviour* | | |
| B1, inflammatory (%) | 9 (45%) | - |
| B2, stricturing (%) | 5 (25%) | - |
| B3, penetrating (%) | 4 (20%) | - |
| Info not available (NA) | 2 | - |
| *Location* | | |
| L1, ileal (%) | 8 (40%) | - |
| L2, colonic (%) | 1 (12%) | - |
| L3, ileocolonic (%) | 8 (23%) | - |
| Info not available (NA) | 3 | - |

| **Disease activity** | | |
|---|---|---|
| HBI, mean±std | 3 ±3 | - |
| Remission, HBI≤4 | 7 (35%) | |
| Mild activity, 5≤HBI≤7 (%) | 1 (5%) | - |
| Moderate activity, 8≤HBI≤15 (%) | 1 (5%) | - |
| Severe activity, 16≤HBI≤∞ (%) | | |
| Info not available (NA) | 11 | - |
| CRP | 6,9±14 | - |

### Results

### Identification of TCRs enriched in CD patients' blood

In CD patients, a group of TCR alpha chains defined by a 15 amino acids CDR3 region and with the gene combination TRAV12-1_TRAJ6 were enriched as compared to healthy controls. The Fisher's exact test confirmed that several clonotypes of the TRAV12-1_TRAJ6 group were present in more CD patients than controls. In detail, 10 clonotypes were significantly enriched after multiple comparison correction and further 3 were nominally significantly enriched. Clonotypes of this TCR group, identified as significantly enriched in CD, share the semi-invariant CDR3 motif CVV**A*GGSYIPTF (SEQ ID NO: 1).

While positions 4 and 5 of the identified motif are the most variable, positions 6 and 7 are more conserved and occupied by A and S amino acids respectively. Moreover, the predicted 3D structure of the TCR alpha CDR3 loop showed that positions 4 and 5 are mostly buried inside the TCR protein structure and may indirectly influence the loop conformation. Positions 6 and 7 are exposed and, together with positions 8 and 11, are predicted to be the most involved into epitope interaction. However, CDR3 loops are very flexible and may adapt to different conformations when interacting with different epitopes, thus, in principle, all CDR3 amino acid positions, excluding the terminal 3-4 residues, may contact the antigen and influence binding affinity.

A higher number of different clonotypes comprising the motive according to the invention (different CDR3 regions) were found in CD patients as compared to healthy controls. In addition, they were more expanded in CD patients' blood and their cumulative abundance (the sum of their relative abundance in each sample) was accordingly significantly higher in CD patients (P-value= 0.008).

The inventors also investigated whether the TCRs according to the invention were associated with phenotypic or clinical traits. No significant association was detected between the cumulative abundance or the number of TCRs according to the invention, and clinical parameters such as sex, age, smoking behaviour, disease location, disease severity or previous treatment for CD. Samples from CD patients were collected prior to start of treatment with anti-TNF biologics. A trend was observed, showing increased numbers of clonotypes comprising the motive according to the invention in patients non-responding to anti-TNF therapy (P-value=0.089). In patients affected by ileal or ileocolonic CD, TCRs according to the invention were more abundant as compared to colonic CD (P-value=0.07).

### Clonotypes comprising the motive according to the invention are present in both blood and intestinal tissue of CD patients

To verify the presence and abundance of TCRs according to the invention in CD, an independent sample collection including matched blood and intestinal tissue of 37 individuals undergoing bowel resection surgery (11 CD, 13 UC and 13 colon cancer (CRC) patients as disease controls was analysed; see Example 2). As in the first collection (Example 1), TCRs according to the invention were confirmed to be increased in number and more abundant in CD patients' blood (P-value=0.002). Clonotypes comprising the motive according to the invention were observed in 9/11(82%) CD, 4/13 (31%) UC and 3/13 (23%) CRC patients' blood. In 8/11 (73%) CD patients, the abundance of TCRs according to the invention was higher than in any other analysed blood sample of the surgery collection. The same trend was observed in intestinal tissue. Clonotypes comprising the motive according to the invention were observed in higher numbers in the blood as compared to the gut. For a subset of patients (4 CD and 5 UC), intestinal mesenteric lymph nodes resected together with the inflamed intestinal tissue were also analysed. While most TCRs according to the invention were observed in the blood, few clonotypes comprising the motive according to the invention were also observed to be present in multiple analysed tissues of the same individual at the same time.

To answer the question whether T-cells comprising the motive according to the invention occur within a particular T cell compartment, blood and intestinal T cells were sorted in CD4+ naïve, CD4+ conventional memory, CD4+ regulatory and CD8+ T cells for 7 CD, 5 UC and 9 CRC additional patients. TCRs according to the invention were found in all analysed fractions, but at different frequencies. Cells according to the invention were most abundant in the CD8+ fraction, particularly in CD patients, with up to 22 TCRs according to the invention detected per individual and higher cumulative abundance as compared to the other cell compartments.

For the three CD patients (Example 7) with a high proportion of cells according to the invention, the CD4+ and CD8+ memory fractions were FACS-purified and single-cell RNA gene expression and TCR sequencing using 10x Genomics' Chromium technology was made. The same analysis was performed for three sex- and age-matched healthy controls.

Cells according to the invention were identified at high abundance (22 to 238 cells per patient) in the three CD patients through their TCRs, while they were rare in healthy controls (0 to 2 CAIT cells per healthy individual). Analysis of TCR alpha/beta pairs showed diverse TCR beta chains associated with the semi-invariant alpha sequences according to the invention. Analysis of the TCR comprising the motive according to the invention alpha/beta chain pairing in clonotypes comprising the motive according to the invention, showed a preferential pairing (38-55% of clonotypes) with beta chains carrying the TRBV7-9 gene in all 3 patients. None of the identified paired TCR beta chains were found in any other individual of the previously analysed bulk TCR datasets (244 whole blood and 58 surgery samples). Thus, these TCR beta sequences seem to be private to single individuals or very rare in the examined population.

### Immunophenotyping of CAIT cells in CD patients and controls

To confirm frequency and phenotype of cells according to the invention identified by TCR and RNA sequencing, we set up an antibody panel for flow cytometry including the antibodies for the TCR V segments TRAV1-2 and TRAV12-1, defining MAIT and CAIT cells respectively, and known unconventional T cell markers such as CD161 (KLRB1) and IL18R. By analysing peripheral blood samples of 20 randomly recruited CD patients and 16 healthy controls, we confirmed that decreased in the blood of CD patients. Cells according to the invention (CD3+TRAV12-1+CD161+IL18R+), were enriched

The frequency difference between patients and controls was observed to be more pronounced in the CD8+ compartment. The same trend for cells according to the invention was observed in the CD4-CD8- and in the CD4+CD8+ fractions.

Using a single-cell transcriptomics approach (see manuscript), it could be showed that the identified T cells seem to have an unconventional innate-like phenotype.

Unconventional T cells are cells not binding the classic MHC alleles but rather MHC-like molecules as MR1 and CD1d. These molecules are less variable among people as compared to classic MHC alleles. Therefore, T cells reactive to MHC-like molecules are also more common among individuals. Therefore, the analysis of these cells and possible drug-discovery research also has higher applicability at population level as compared to a conventional T cell which may be rather private of single individuals.

We also performed a flow cytometry analysis of the T cells. A flow cytometry analysis looks at the protein markers expressed on the surface of the cells and helps to identify the cell phenotype and function. It was found that the cell population including the clonotypes is increased in both CD and UC patients as compared to healthy controls. In the analysis, the cell population was defined to be CD161+TRAV12-1+, where TRAV12-1 is the part of the TCR protein encoded by the TRAV12-1 variable gene.

## Claims

1. A protein comprising an amino acid sequence according to SEQ ID NO: 1.

2. The protein of claim 1, wherein the amino acid in position 4 of SEQ ID NO: 1 is selected from the group consisting of N, S, R, T and I, preferably consisting of N, S and R and/or wherein the amino acid in position 5 of SEQ ID NO: 1 is selected from the group consisting of R, V, L, F, M, I, H, S, T, A, P and G.

3. The protein of claim 1 or claim 2, wherein the amino acid in position 7 of SEQ ID NO: 1 is A or S, wherein A is preferred.

4. The protein of any of claims 1 to 3, wherein the amino acid sequence according to SEQ ID NO: 1 is selected from the group consisting of amino acid sequences SEQ ID NO: 2 to 295.

5. The protein of any of claims 1 to 4, wherein the protein is a TCR.

6. The protein of claim 5, wherein the amino acid sequence according to SEQ ID NO: 1 is comprised in the alpha chain of the TCR.

7. The protein of claim 6, wherein the amino acid sequence according to SEQ ID NO: 1 is comprised in the in the CDR3 region.

8. A T-cell, comprising the protein of any of claims 1 to 8.

9. The T-cell of claim 8, wherein the T-cell is a human T-cell.

10. The T-cell of claim 8 or 9, wherein the T-cell is a CD4+ cell and/or a CD8+ cell and/or a CD161+ cell, preferably a CD8+ cell.

11. A nucleotide sequence encoding for an amino acid sequence according to sequence ID NO: 1, preferably according to any of amino acid sequences SEQ ID NO: 2 to 295, wherein the nucleotide sequence more preferably is selected from the DNA sequences SEQ ID NO: 296 to 1205, or an mRNA sequence encoded by a DNA sequence according to any of DNA sequence SEQ ID NO: 296 to 1205.

12. A use of a protein according to any of claims 1-7 or a use of a T-cell according to any of claims 8-10 or a use of an mRNA according to claim 11 in the research for a medicament for treatment of a chronic inflammatory disease preferably inflammatory bowel disease (IBD).

13. The use of claim 12, wherein the chronic inflammatory disease is IBD, preferably Crohn's disease (CD) or ulcerative colitis (UC), preferably CD, more preferably ileal CD and/or ileocolonic CD.

14. A use of a protein according to any of claims 1-7 or a use of a T-cell according to any of claims 8-10 or a use of an mRNA according to claim 11 in the development of an antibody directed against the protein and/or the T-Cell or the mRNA.

15. The use of claim 14, wherein the antibody is directed against an epitope, comprising at least a part of the amino acid sequence according to SEQ ID NO: 1, wherein preferably the amino acid sequence according to SEQ ID NO: 1 is further defined as in any of claims 2-4.

16. An Antibody directed against an epitope, comprising at least a part of the amino acid sequence according to SEQ ID NO: 1, wherein preferably the amino acid sequence according to SEQ ID NO: 1 is further defined as in any of claims 2-4 and/or wherein the Antibody is a monoclonal antibody.
